# EUROPEAN PATENT SPECIFICATION

(11) **EP 1 860 198 B1**
(45) Date of publication and mention of the grant of the patent: **10.11.2010**
(21) Application number: 07100033.5
(22) Date of filing: 02.01.2007
(51) Int. Cl.: C12Q 1/68

(54) **Method and apparatus for concentrating and amplifying nucleic acid in single micro chamber**
Verfahren und Vorrichtung zur Konzentration und Amplifikation von Nukleinsäure in einer Einzelmikrokammer
Procédé et appareil de concentration et d'amplification d'acide nucléique dans une seule microchambre

(30) Priority: 22.05.2006 KR 20060045812
(43) Date of publication of application: 28.11.2007
(73) Proprietor: Samsung Electronics Co., Ltd., Suwon-si, Gyeonggi-do (KR)
(72) Inventor: Kim, Young-rok c/o Samsung Adv. Inst. of Techn., Yongin-si Gyeonggi-do (KR); Min, Jun-hong c/o Samsung Adv. Inst. of Techn., Yongin-si Gyeonggi-do (KR); Lee, In-ho c/o Samsung Adv. Inst. of Techn., Yongin-si Gyeonggi-do (KR); Lee, Young-sun c/o Samsung Adv. Inst. of Techn., Yongin-si Gyeonggi-do (KR); Yoo, Chang-eun c/o Samsung Adv. Inst. of Techn., Yongin-si Gyeonggi-do (KR); Han, Ki-woong c/o Samsung Adv. Inst. of Techn., Yongin-si Gyeonggi-do (KR)
(74) Representative: Grünecker, Kinkeldey, Stockmair & Schwanhäusser Anwaltssozietät

(56) References cited:
- WO-A-2004/061085
- US-A1- 2005 142 570
- LIU W-T ET AL: "Environmental microbiology-on-a-chip and its future impacts" TRENDS IN BIOTECHNOLOGY, ELSEVIER PUBLICATIONS, CAMBRIDGE, GB, vol. 23, no. 4, April 2005 (2005-04), pages 174-179, XP004797077 ISSN: 0167-7799
- PANARO N J ET AL: "Micropillar array chip for integrated white blood cell isolation and PCR" BIOMOLECULAR ENGINEERING, ELSEVIER, NEW YORK, NY, US, vol. 21, no. 6, February 2005 (2005-02), pages 157-162, XP004770258 ISSN: 1389-0344
- SE HO PARK ET AL: "Cylindrical pillars in silicon PCR chip enhance the performance of DNA amplification" SOLID-STATE SENSORS, ACTUATORS AND MICROSYSTEMS, 2005. DIGEST OF TECHNICAL PAPERS. TRANSDUCERS '05. THE 13TH INTERNATIONAL CONFERENCE ON SEOUL, KOREA JUNE 5-9, 2005, PISCATAWAY, NJ, USA,IEEE, 5 June 2005 (2005-06-05), pages 1604-1607, XP010828796 ISBN: 0-7803-8994-8
- SAMPER V ET AL: "Microfluidic sample preparation for nucleic acid analysis" SEMICONDUCTOR CONFERENCE, 2004. CAS 2004 PROCEEDINGS. 2004 INTERNATIONAL SINAIA, ROMANIA 4-6 OCT. 2004, PISCATAWAY, NJ, USA,IEEE, US, vol. 1, 4 October 2004 (2004-10-04), pages 51-58, XP010775915 ISBN: 0-7803-8499-7
- ZHANG ET AL: "PCR microfluidic devices for DNA amplification" BIOTECHNOLOGY ADVANCES, ELSEVIER PUBLISHING, BARKING, GB, vol. 24, no. 3, 2 December 2005 (2005-12-02), pages 243-284, XP005362290 ISSN: 0734-9750
- CHENG J ET AL: "CHIP PRC II. INVESTIGATION OF DIFFERENT PCR AMPLIFICATION SYSTEMS IN MICROFABRICATED SILICON-GLASS CHIPS" NUCLEIC ACIDS RESEARCH, OXFORD UNIVERSITY PRESS, SURREY, GB, vol. 24, no. 2, 1996, pages 380-385, XP000578332 ISSN: 0305-1048
- HODKO D ET AL: "DETECTION OF PATHOGENS USING ON-CHIP ELECTROCHEMICAL ANALYSIS OF PCR AMPLIFIED DNA MOLECULES" PROCEEDINGS OF THE SPIE, SPIE, BELLINGHAM, VA, US, vol. 4265, 25 January 2001 (2001-01-25), pages 65-74, XP008000573 ISSN: 0277-786X
- KIM ET AL: "Fabrication and characterization of a PDMS-glass hybrid continuous-flow PCR chip" BIOCHEMICAL ENGINEERING JOURNAL, ELSEVIER, AMSTERDAM, NL, vol. 29, no. 1-2, 1 April 2006 (2006-04-01), pages 91-97, XP005335973 ISSN: 1369-703X
- PAEGEL B M ET AL: "Microfluidic devices for DNA sequencing: sample preparation and electrophoretic analysis" CURRENT OPINION IN BIOTECHNOLOGY, LONDON, GB, vol. 14, no. 1, February 2003 (2003-02), pages 42-50, XP002381499 ISSN: 0958-1669
- HOURFAR M K ET AL: "High-throughput purification of viral RNA based on novel aqueous chemistry for nucleic acid isolation" CLINICAL CHEMISTRY, AMERICAN ASSOCIATION FOR CLINICAL CHEMISTRY, WASHINGTON, DC, US, vol. 51, no. 7, July 2005 (2005-07), pages 1217-1222, XP002403248 ISSN: 0009-9147
- "Hydrogen bond" Retrieved from the Internet: URL:http://en.wikipedia.org/wiki/Hydrogen_ bond>
- "Hydrophile" Retrieved from the Internet: URL:http://en.wikipedia.org/wiki/Hydrophil e>

<110> Samsung Electronics Co., Ltd.
<120> Method and apparatus for concentrating and amplifying a nucleic acid in a single micro chamber
<130> EP46154FZ106pau
<140> not yet assigend
   <141> herewith
<150> KR 10-2006-0045812
   <151> 2006-05-22
<160> 2
<170> KopatentIn 1.71
<210> 1
   <211> 20
   <212> DNA
   <213> Artificial sequence
<220>
   <223> forward primer
<400> 1
   yccakactcc tacgggaggc 20
<210> 2
   <211> 22
   <212> DNA
   <213> Artificial sequence
<220>
   <223> reverse primer
<400> 2
   gtattaccgc rrctgctggc ac 22

## Description

### BACKGROUND OF THE INVENTION

### 1. Field of the Invention

The present invention relates to a method for concentrating and amplifying a nucleic acid in a single micro chamber.

### 2. Description of the Related Art

The production of high purity double-stranded plasmid DNAs, single-stranded phage DNAs, chromosomal DNAs, and agarose gel-purified DNA fragments is very important in molecular biology. Ideal methods of purifying DNAs should be simple and quick, and include little additional manipulation of samples. DNAs obtained using such methods can be used for direct transformation, restriction enzyme analysis, ligation, or sequencing. Such methods are very attractive in the automated production of DNA samples, which is favored in research and diagnosis labs.

Conventionally, a method of purifying nucleic acid using a solid phase is known. For example, U.S. Patent No. 5,234,809 discloses a method of purifying nucleic acid using a solid phase to which nucleic acids are bound, the method including: mixing a starting material, a chaotropic material, and a nucleic acid binding solid phase; separating the solid phase with the nucleic acid bound thereto from the liquid, and washing the solid phase nucleic acid complexes. However, this method is time consuming and complicated, and thus is not suitable for a Lab-On-a-Chip (LOC). The method also has a problem in that a chaotropic material has to be used.

U.S. Patent No. 6,291,166 discloses a method of archiving nucleic acid using a solid-phase matrix. Since nucleic acids are irreversibly bound to a solid-phase matrix, this method is advantageous in that a delayed analysis or repeated analysis of the nucleic acid solid-phase matrix complex after storage is possible. However, according to this method, alumina, which has a positively charged surface, needs to be rendered hydrophilic by addition of basic materials, such as NaOH, and nucleic acids are irreversibly bound to the hydrophilic alumina, and thus cannot be separated from the alumina. Accordingly, the method has a problem in that Polymerase Chain Reaction (PCR) yield is low in a solid-phase matrix to which DNAs are irreversibly bound.

U.S. Patent No. 6,383,783 discloses a method of isolating nucleic acid from a sample, the method including: introducing a sample containing target nucleic acids into a hydrophobic organic polymer solid-phase in order to attach a target nucleic acid to the solid-phase; and adding a non-ionic surfactant to the solid-phase and removing the attached target nucleic acid. However, while nucleic acids are separated using a hydrophobic solid-phase in the conventional method, concentration and amplification of nucleic acids are sequentially performed in a single micro chamber using a solid-phase having a hydrophilic surface and a kosmotropic salt in the present invention.

LIU W-T ET AL: "Environmental microbiology-on-a-chip and its future impact,. TRENDS IN BIOTECHNOLOGY. ELSEVIER PUBLICATIONS. CAMBRIDGE. GB, vo. 23, no. 4, April 2005(2005-04),_pages 174-179 discloses laboratory-on-a-chip (LOC) devices comprising wells as fluid reservoirs that are connected via microchannels with chambers and their applications in performing sample preparation together with biochemical reactions such as PCR, and detection steps.

WO 2004/061085 A discloses methods and apparatuses for implementing microfluidic analysis devices having fluid control structures that can be used to implement a pathogen detection and analysis system including immunoaffinity capture and analysis, such as PCR and capillary electrophoresis.

HOURFAR M K ET AL: "High-throughput purification of viral RNA based on novel aqueous chemistry for nucleic acid isolation", CLINICAL CHEMISTRY, AMERICAN ASSOCIATION FOR CLINICAL CHEMISTRY, WASHINGTON. DC, US, vol. 51, no. 7, July 2005 (2005-07), pages 1217-1222 discloses extraction of viral nucleic acids from plasma samples by use of common magnetic silica beads, wherein nucleic acids are bound to the beads at acidic conditions in the presence of a kosmotropic salt and_are eluted at a slightly alkaline pH.

The inventors of the present invention studied a method of concentrating and amplifying a nucleic acid based on the prior art as described above, and confirmed that concentration and amplification of a nucleic acid can be sequentially performed in a single micro chamber when adding a kosmotropic salt to a micro chamber having a hydrophilic functional group on its surface. Thereafter, nucleic acids can be concentrated in the micro chamber, and PCR can be performed in the same micro chamber using the concentrated nucleic acid.

### SUMMARY OF THE INVENTION

The present invention provides a method of sequentially performing nucleic acid concentration and amplification in a single chamber, which can minimize sample losses, time consumption and costs.

### BRIEF DESCRIPTION OF THE DRAWINGS

The above and other features and advantages of the present invention will become more apparent by describing in detail exemplary embodiments thereof with reference to the attached drawings in which:
FIG. 1 is a view illustrating how concentration and amplification of nucleic acid are performed in a single micro chamber, according to an embodiment of the present invention;
FIG. 2 is a view illustrating how nucleic acid is bound on a hydrophilic surface of a micro chamber in the presence of a kosmotropic salt, according to an embodiment of the present invention;
FIG. 3 is a graph showing polymerase chain reaction (PCR) efficiency subject to an increase in a chip surface area;
FIG. 4 illustrates two graphs showing PCR efficiency with respect to PEG concentration;
FIG. 5 illustrates two graphs showing PCR efficiency with respect to BSA concentration.
FIG. 6 illustrates photos representing six kinds of chips having different surface areas.
FIG. 7 is a graph showing DNA binding efficiency and PCR efficiency of the 6 chips having different surface areas .
FIG. 8 is a graph showing the increase in PCR efficiency subject to nucleic acid concentration, according to an embodiment of the present invention.
FIG. 9 is a standard curve showing Ct values subject to the copy number of target genes of *E. coli* BL21 gDNA. The Ct values of tested chips (Chip #2 and Chip #6) after nucleic acid concentration were assigned to their corresponding position on the standard curve.
FIG. 10 is a graph showing the increase in PCR efficiency subject to nucleic acid concentration and purification from cell lysate, according to an embodiment of the present invention.
FIG. 11 is a standard curve showing Ct values subject to the number of *E*. *coli* BL21 cells. The Ct value of the tested chip (Chip #2) after nucleic acid concentration was assigned to its corresponding position on the standard curve.

### DETAILED DESCRIPTION OF THE INVENTION

The present invention will now be described in more detail with reference to the accompanying drawings, in which exemplary embodiments of the invention are shown.

According to an embodiment of the present invention, there is provided a method of sequentially concentrating and amplifying a nucleic acid in a single chamber, the method comprising: introducing a nucleic acid-containing sample and a solution comprising a kosmotropic salt, or a mixture of said sample and said solution, into a micro chamber having a hydrophilic surface, concentrating the nucleic acid by binding the nucleic acid to the surface of the micro chamber, adding a PCR mixture to the chamber, and amplifying the nucleic acid by performing PCR.

The present invention relates to a technique for preparing a sample for nucleic acid analysis and performing real-time PCR in a single micro chamber. To amplify and detect target genes, DNA has to be isolated and purified from a biological sample. Various impurities that are present in a cell lysate and which inhibit PCR are removed by a purification step. In a conventional process, purification, amplification and detection of specific genes are each performed in a separate micro chamber so that time and costs are extensively consumed in gene analysis, and the possibility of cross contamination of a sample is high. In the present invention, nucleic acid purification/concentration and nucleic acid amplification are performed in the same chamber, and thus analysis processes are simplified and the risk of cross contamination is reduced.

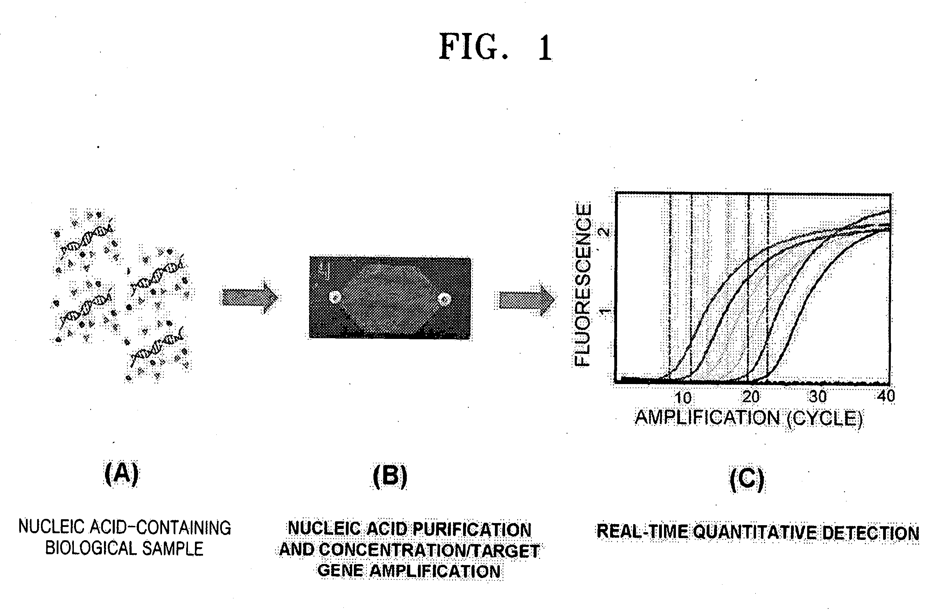

FIG. 1 is a view illustrating how concentration and amplification of a nucleic acid are performed in a single micro chamber, according to an embodiment of the present invention. A target gene is purified and concentrated from a biological sample including the nucleic acid in a single micro chamber. Then, the purified and concentrated target gene is amplified in the same micro chamber and can be detected in real time. However, several requirements have to be satisfied to achieve this. First, reagents used in DNA purification and concentration should not affect the subsequent real-time polymerase chain reaction (PCR), and therefore PCR inhibitors such as a chaotropic salt and ethanol cannot be used. Second, PCR has to be performed in a SiO₂ chip having a large surface area. However, a SiO₂-surface acts as a PCR inhibitor. Third, the SiO₂ chip should have a high DNA binding efficiency.

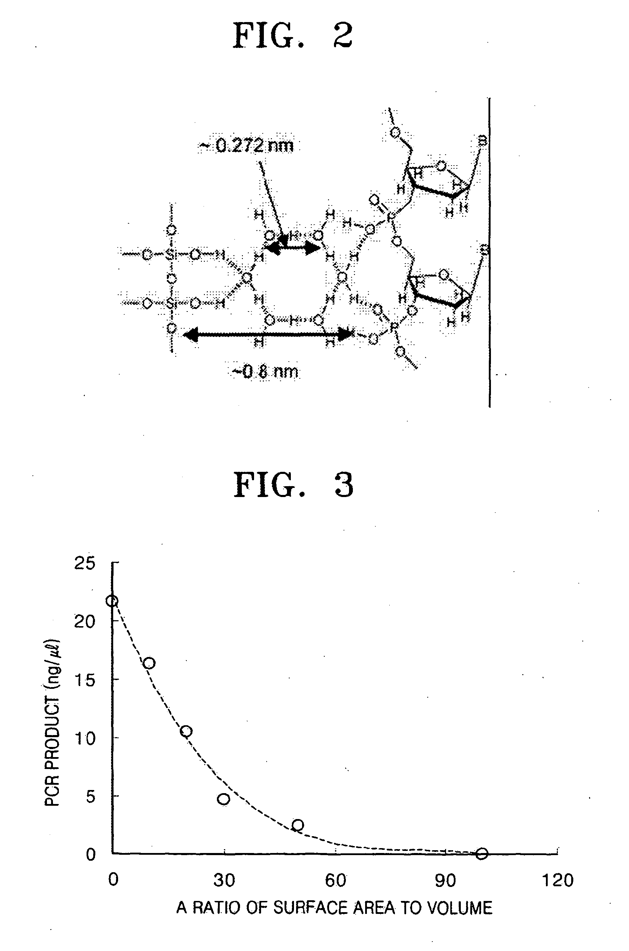

To solve these problems, a kosmotropic salt is used instead of a chaotropic salt that is conventionally used in the purification of nucleic acids. When a chaotropic salt is used, the surface of a micro chamber is dehydrated and nucleic acids is directly bound to the micro chamber via hydrogen bonding. Such binding of nucleic acids are affected by pH. Moreover, a chaotropic salt acts as a PCR inhibitor. Contrary thereto, when a kosmotropic salt is used, the surface of the micro chamber is hydrated and a stabilized water layer is formed on the surface of the micro chamber. Nucleic acids can thus be hydrated onto the surface of the micro chamber resulting from a salting-out effect by hydrophilic interaction. FIG. 2 illustrates how a nucleic acid is bound via a water layer to a hydrophilic surface of a micro chamber in the presence of a kosmotropic salt, according to an embodiment of the present invention. As can be seen in FIG.2, due to the salting-out effect of a kosmotropic salt, a silica substrate (left part of FIG. 2) is covered by a water layer, which is bound to the silica substrate by hydrogen bonding. The formed water layer (middle part of FIG. 2) forms a further hydrogen bond with the nucleic acid (right part of FIG. 2). Thus, the nucleic acid is bound to the micro chamber surface by means of the stabilized water layer. Therefore, nucleic acid binding to the hydrophilic micro chamber does not require the acidic condition that is conventionally required for nucleic acid binding, and the surface of the micro chamber need not be limited to silica.

Concentrating a nucleic acid is initiated, according to an embodiment of the present invention, when a sample containing a nucleic acid and a solution containing a kosmotropic salt, or a mixture thereof, are introduced into a micro chamber. Thereupon, water forms a hydrogen bond with the surface of the micro chamber due to the salting-out effect of a kosmotropic salt, and then the water layer formed on the surface, in turn, forms a hydrogen bond with the nucleic acid. As a result, the nucleic acid is bound and concentrated to the surface of the micro chamber via the water layer. By washing off other components, which are not bound to the surface of the chamber, contrary to the bound nucleic acid, the nucleic acid can be purified and concentrated in a purer form. A solution used for nucleic acid binding to the micro chamber can also be used as a washing solution. Preferably, the concentration of the kosmotropic salt in the washing solution is lower than the concentration used for binding.

For amplifying a nucleic acid according to the current embodiment of the present invention, a PCR mixture is added to a micro chamber, in which the nucleic acid is concentrated, and then PCR is performed using a target nucleic acid as template that is bound to the surface of the micro chamber. Alternatively, the target nucleic acid can be isolated from the surface of the micro chamber after purifying the nucleic acid and before performing PCR. It will be understood by those skilled in the art that the composition and concentrations of the PCR mixture may vary depending on the polymerase used. In addition, the PCR conditions can be appropriately selected according to the length of the PCR product to be amplified, the sequence homology between the template and the primer, the length of the primer and the like.

In the method of concentrating and amplifying a nucleic acid according to the current embodiment of the present invention, polyethylene glycol (PEG) and/or bovine serum albumin (BSA) can be added before performing the PCR. A surface consisting of SiO₂ acts as a PCR inhibitor in a micro chamber, and thus a proper additive needs to be added to reduce this inhibition. Therefore, in an embodiment of the present invention, PCR amplification efficiency is increased by adding polyethylene glycol (PEG) and/or bovine serum albumin (BSA) to the micro chamber. The concentration of the polyethylene glycol (PEG) and the bovine serum albumin (BSA) may be 1-10 % by volume and 0.1-10 mg/ml, respectively. When the concentration of PEG and BSA is beyond these ranges, PCR amplification efficiency is decreased.

In the method of concentrating and amplifying a nucleic acid according to the current embodiment of the present invention, the surface of the micro chamber may have a plurality of pillars. To increase the probability of contacting a nucleic acid contained in a sample with the micro chamber surface, the surface of the micro chamber has preferably a pillar structure providing a large surface area, rather than a planar structure. The pillars can have various shapes, such as a square, rectangle, diamond, cylinder, cone, hexagon, octagon and the like. An distance between the pillars may be 8 to 50 µm. When the distance between the pillars is beyond this range, the efficiency of binding and amplifying the nucleic acid decreases.

In the method of concentrating and amplifying a nucleic acid according to the current embodiment of the present invention, the hydrophilic functional group of the micro chamber can be any hydrophilic group such as a hydroxyl group, an amine group, a carboxyl group, a polycarboxyl group, a silanol group or the like.

In the method of concentrating and amplifying a nucleic acid according to the current embodiment of the present invention, the kosmotropic salt can be sulfate (SO₄²⁻), hydrogen phosphate (HPO₄²⁻), hydroxide (OH⁻), fluoride (F⁻), formate (HCOO-), acetate (CH₃COO⁻), etc., but is not limited thereto. The kosmotropic salt induces protein crystallization, acts as a salting-out ion for a hydrophobic particle and forms a structure of water according to the Hofmeister series.

In the method of concentrating and amplifying a nucleic acid according to the current embodiment of the present invention, the sample containing a nucleic acid and the solution comprising a kosmotropic salt may have a pH of 3 to 10. When the pH of the mixture of the sample containing nucleic acid and the solution comprising a kosmotropic salt, or a mixture thereof, is beyond this range, DNA can be physically and chemically denatured and subsequent processes can be affected.

In the method of concentrating and amplifying a nucleic acid according to the current embodiment of the present invention, the concentration of the kosmotropic salt can be 100 to 2,000 mM. When the concentration of the kosmotropic salt is less than 100 mM, the efficiency of binding the nucleic acid to the micro chamber is decreased. When the concentration of the kosmotropic salt is greater than 2,000 mM, it is difficult to prepare the solution.

In the method of concentrating and amplifying a nucleic acid according to the current embodiment of the present invention, the sample containing the nucleic acid can be blood, serum, urine, saliva or cell lysate of bacteria existing in a cell culture fluid, but is not limited thereto. The sample containing the nucleic acid according to an embodiment of the present invention may be from a mammal, a plant, bacteria, or yeast. The sample can be in a single cell form or tissue form, and the cell or tissue may stem from cultures in vitro.

In the present application, there is provided an apparatus for sequentially performing concentration and amplification of a nucleic acid, the apparatus comprising: a micro chamber having a hydrophilic functional group on its surface; a nucleic acid-containing sample storing part, which is fluidically connected to the micro chamber, for providing the micro chamber with the nucleic acid-containing sample; a kosmotropic salt solution storing part, which is fluidically connected to the micro chamber, for providing the micro chamber with a kosmotropic salt; a PCR mixture storing part, which is fluidically connected to the micro chamber, for providing the micro chamber with a PCR mixture; and a heating part and a cooling part, i.e. a temperature control means for heating and cooling the micro chamber.

The apparatus for concentrating and amplifying a nucleic acid essentially comprises a micro chamber having a hydrophilic functional group on its surface, a nucleic acid-containing sample storing part, a kosmotropic salt solution storing part, a PCR mixture storing part and a heating and cooling part. In the apparatus for concentrating and amplifying a nucleic acid the micro chamber comprises a chamber in a chip, and has a hydrophilic functional group on its surface so that water forms a water layer on the surface of the chamber by hydrogen bonding. The stable water layer formed binds with nucleic acid via hydrogen bonding. The hydrophilic functional group can be any hydrophilic group such as a hydroxyl group, an amine group, a carboxyl group, a polycarboxyl group, a silanol group or the like.

The nucleic acid-containing sample storing part is a part that provides the micro chamber with the nucleic acid-containing sample. Said sample storing part is preferably connected to the micro chamber by means of a microchannel.

The kosmotropic salt solution storing part is a part that provides the micro chamber with the kosmotropic salt. Said solution storing part is preferably connected to the micro chamber by means of a microchannel. When a sample containing a nucleic acid to be isolated is introduced in the apparatus, the kosmotropic salt solution storing part provides the micro chamber with the kosmotropic salt. The nucleic acid-containing sample and the kosmotropic salt are mixed in the apparatus, and the nucleic acid is bound to the micro chamber due to a salting-out effect of the kosmotropic salt.

The PCR mixture storing part is a part that provides the micro chamber with the PCR mixture. The PCR mixture storage part is preferably connected to the micro chamber by means of a microchannel. After the nucleic acid is concentrated in the micro chamber, the PCR mixture storing part injects the PCR mixture into the micro chamber to perform PCR.

The temperature control means heats and cools the micro chamber. When the PCR mixture is introduced in the micro chamber, the temperature control means controls the temperature of the micro chamber by heating and cooling according to the PCR conditions. The temperature control means may comprise a combined heating and cooling part or an individual heating part and an individual cooling part.

In the apparatus for concentrating and amplifying a nucleic acid , the surface of the micro chamber may have a plurality of pillars. To increase the probability of contacting a nucleic acid contained in a sample with the micro chamber, the surface of the micro chamber has preferably a pillar structure having a large surface area rather than a planar structure. The pillars can have various shapes, such as a square, rectangle, diamond, cylinder, cone, hexagon, octagon or the like. An distance between the pillars may be 8 to 50 µm. When the distance between the pillars is beyond this range, the efficiency of binding and amplification the nucleic acid decreases.

In the apparatus for concentrating and amplifying a nucleic acid , the sample containing the nucleic acid can be blood, serum, urine, saliva or cell lysate of bacteria existing in a cell culture fluid, but is not limited thereto. The sample containing the nucleic acid according to an embodiment of the present invention may be from a mammal, a plant, bacteria, or yeast. The sample can be in a single cell form or tissue form, and the cell or tissue may stem from cultures in vitro.

In the present application, there is provided an apparatus for sequentially performing concentration and amplification of a nucleic acid, the apparatus comprising: a chip including a micro chamber having a hydrophilic functional group on its surface; and a nucleic acid amplification part on which the chip is mounted. The apparatus for concentrating and amplifying a nucleic acid comprises a chip and a nucleic acid amplification part. The chip includes the micro chamber having the hydrophilic functional group on its surface. Preferably, the chip comprises a single chamber. The micro chamber has the hydrophilic functional group on its surface so that, when a nucleic acid-containing sample and a solution comprising kosmotropic salt are introduced into the micro chamber, the nucleic acid is bound to the surface of the micro chamber and the nucleic acid is concentrated. The nucleic acid amplification part includes the chip thereon and amplifies nucleic acid concentrated in the micro chamber of the chip. The chip including the micro chamber, in which a nucleic acid is concentrated, is placed in the nucleic acid amplification part, a PCR mixture is introduced into the micro chamber, and then PCR is performed in the micro chamber of the chip to amplify the concentrated nucleic acid.

In the present application, there is provided a lab-on-a-chip including the apparatus for concentrating and amplifying a nucleic acid. In the apparatus for concentrating and amplifying a nucleic acid-, each functional element can be implemented by process-on-a-chip using a known microfuidics technique and a micro-electro-mechanical system (MEMS) device, and can be further implemented by a lab-on-a-chip.

Hereinafter, the present invention will be described in further detail with reference to the following examples. These examples are for illustrative purposes only and are not intended to limit the scope of the present invention.

### Example 1: PCR efficiency according to an increase in the surface area of a chip

PCR efficiency was determined according to an increase in the surface area of a chip. PCR was performed using a TMC1000 (Samsung) instrument. The TMC1000 is an instrument that performs quantitative PCR on a silicon-based chip. Unless otherwise specified, the template DNA used in all the examples is a genome DNA of *E*. *coli* BL21, 1.4 × 10⁵ copies of which were used per reaction. A copy of the genome DNA has seven target nucleic acids (16s-rRNA gene), and thus a total of 10⁶ copies of target gene per reaction are included. A forward primer (YCCAKACTCCTACGGGAGGC; SEQ ID NO: 1) and a reverse primer (GTATTACCGCRRCTGCTGGCAC; SEQ ID NO: 2) were used as PCR primers.

Unless otherwise specified, the PCR mixture comprises 1× PCR buffer (Solgent Co. Ltd.), 5.0 mM of MgCl₂, 200 µM of dNTP, 0.2 µM of each primer, 0.1 mg/ml of BSA, and 0.1 U/µl of Taq DNA polymerase. The temperature profile of the PCR is as follows. PCR in an Eppendorf PCR tube was performed at 94 °C for 5 minutes in an initial cycle, and at 94 °C for 30 seconds, at 62 °C for 30 seconds and at 72_°C for 30 seconds in 30 cycles. Then, the obtained PCR product was quantified using a Bioanalyzer (Agilent). PCR in a chip was performed at 94 °C for 1 minute in an initial cycle, and at 94 °C for 5 seconds, at 62 °C for 5 seconds and at 72 °C for 20 seconds in 40 cycles. Then, the obtained PCR product was monitored in real time using the TMC1000 (Samsung) instrument.

FIG. 3 is a graph showing the PCR efficiency according to an increase in the chip's surface area. Referring to FIG. 3, it can be seen that the higher the surface area to volume ratio of a chip is, the less the amount of the PCR product obtained. Thus, the amount of the produced PCR product is closely related to the surface area to volume ratio of the chip. To perform PCR using a silicone-based chip with a large surface area, additives are required that reduce the PCR inhibition caused by the silicone-surface.

### Example 2: PCR efficiency according to the PEG concentration

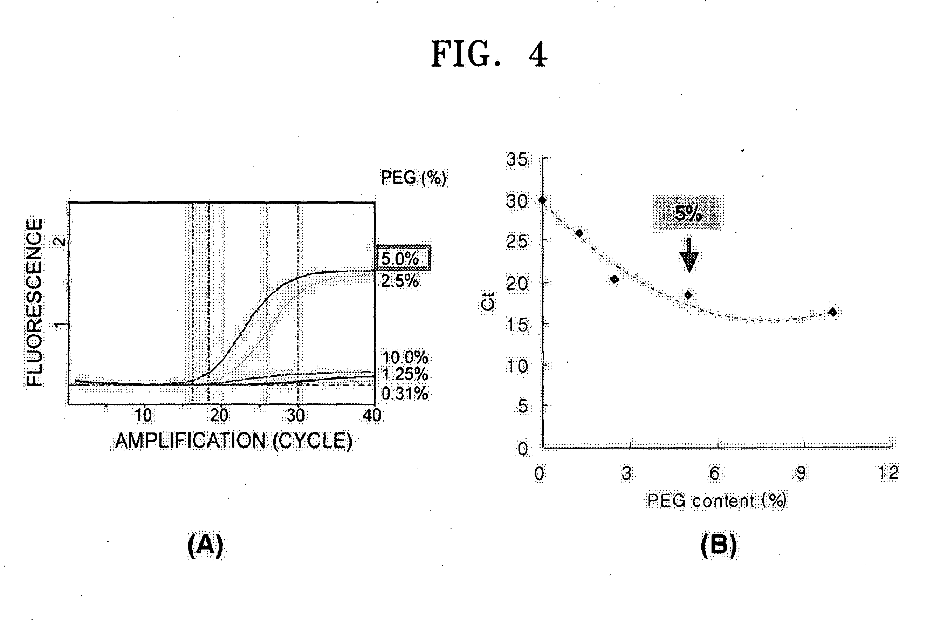

PCR efficiency according to the concentration of polyethylene glycol (PEG) with respect to a chip having a large surface area was determined. PCR was performed in the same manner as in Example 1 except that 5 samples were prepared by adding concentrations of 0.31 %-wt, 1.25 %-wt, 2.5 %-wt, 5 %-wt and 10 %-wt of PEG, respectively, to a PCR mixture. FIG. 4 illustrates two graphs showing PCR efficiency with respect to the PEG concentration. In FIG. 4, the graph on the left refers to Rn values, and the graph on the right refers to fluorescence measurement values Ct. The fluorescence measurement value Ct is the threshold cycle indicating the number of cycles, when the amount of PCR product reaches a threshold fluorescence signal. When the same sample is used, the lower a Ct value is, the higher is the PCR efficiency. Ct is a unit indicating that 1 cycle difference denotes doubling or halving DNA concentration. Rn is related to the concentration of the PCR product obtained after performing the PCR. The higher an Rn value is, the higher is the concentration of the PCR product obtained. As shown in FIG. 4, when 5 % (w/w) of PEG is added, Rn has the highest value, and Ct has the lowest value, thus obtaining the highest PCR efficiency with 5 % (w/w) PEG.

### Example 3: PCR efficiency according to the concentration of BSA

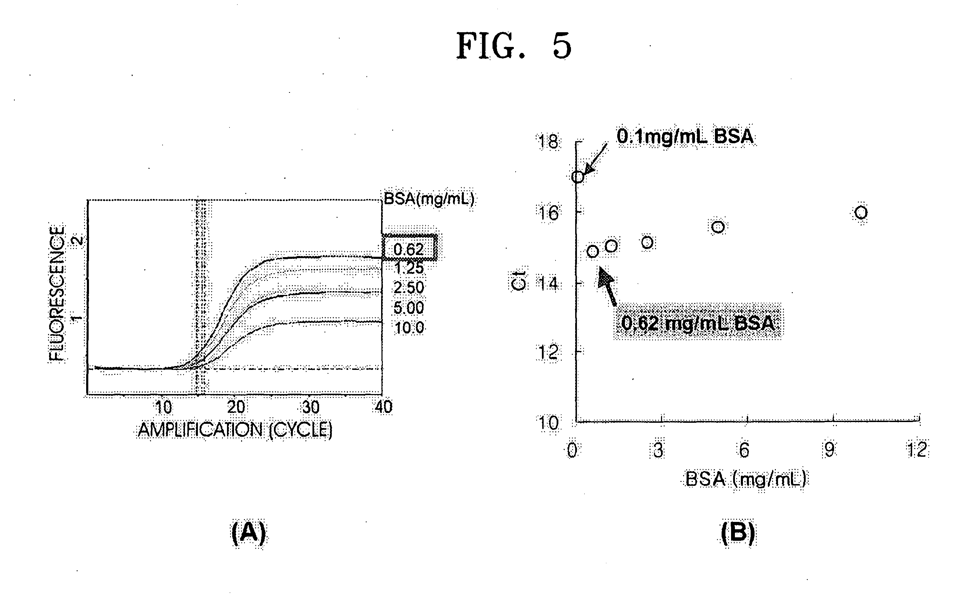

PCR efficiency according to the concentration of bovine serum albumin (BSA) with respect to a chip having a large surface area was determined. PCR was performed in the same manner as in Example 1, except that 5 % of PEG was added to the PCR mixture, and 5 samples were prepared by adding amounts of 0.62 mg/ml, 1.25 mg/ml, 2.5 mg/ml, 5 mg/ml and 10 mg/ml of BSA thereto. FIG. 5 illustrates two graphs showing PCR efficiency subject to the BSA concentration. In FIG. 5, the graph on the left panel refers to Rn values, and the graph on the right refers to Ct values. As shown in FIG. 5, when 0.62 mg/ml of BSA is added, Rn has the highest value, and Ct has the lowest value, thus obtaining the optimum PCR efficiency with 0.62 mg/ml of BSA.

### Example 4: PCR efficiency according to chips having various surface areas

To determine PCR efficiency according to chips having various surface areas, 6 kinds of chips were prepared. A process of preparing a silicon-based chip (Si/SiO₂) is as follows:
(1) Wafer washing
   A wafer was treated in a Piranha solution (H₂SO₄:H₂O₂=3:1, 120 °C) for 15 minutes, and was dried after washing under running water.
(2) Hexamethyldisilazane (HMDS) coating
   5 ml of hexamethyldisilazane (HMDS) was coated on the washed wafer using a spin coater. Coating was performed at 500 rpm for 5 seconds and at 4000 rpm for 40 seconds, and then the coated wafer was baked in a hot plate at 120 °C for two minutes.
(3) Photoresist (PR) coating
   5 ml of photoresist (GXR 601) was coated on the baked wafer, and then coating was performed at 500 rpm for 5 seconds and at 4000 rpm for 40 seconds.
(4) Soft baking
   The PR-coated wafer was baked at 95 °C for two minutes using a hot plate.
(5) UV exposure
   A mask for manufacturing the respective pillar pattern was installed in a UV aligner (i-line), and then 250 mJ of UV light was irradiated through the mask onto the soft baked wafer.
(6) Development
   Development was performed using a MIF 300 developer.
(7) Hard baking
   The developed wafer was hard baked at 115 °C for two minutes.
(8) Deep reactive ion etching (RIE)
   The hard baked wafer was etched with 100 µm of Si using a STS ICP-RIE device.
(9) Ashing
   The photoresist was ashed from the etched wafer using an Asher device.
(10) PR strip
   The ashed wafer was treated in a Piranha solution for 15 minutes, washed and dried in order to remove and wash the remaining PR.
(11) Hydrogen Fluoride (HF) process
   The resulting wafer was treated with diluted HF for one minute, and the natural oxide was removed.
(12) Si oxidation
   For SiO₂ development, thermal wet oxidation was performed using vapor to develop a thickness of 3000 Å.
(13) Wafer washing
   The wafer was treated in a Piranha solution for 15 minutes, washed and dried.
(14) Anodic bonding
   A glass substrate was placed on the washed wafer obtained in (13) and 400 °C and a voltage of 1000 volts were applied thereto for binding the glass substrate to the wafer to complete a microchip.

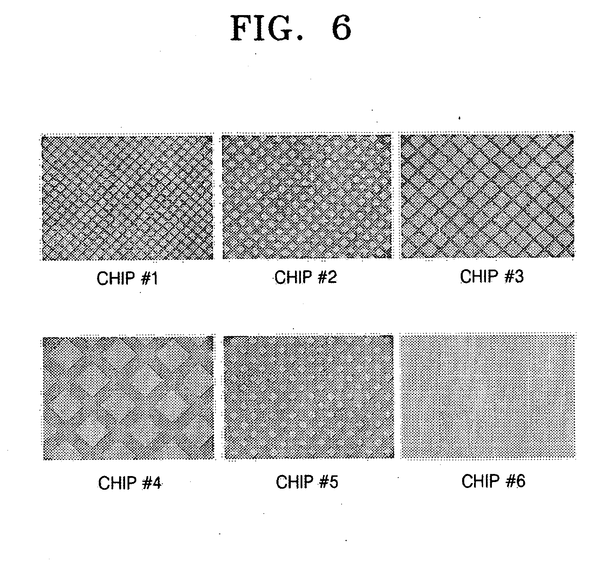

FIG. 6 illustrates photos representing six types of chips having different surface patterns. Pillar size, the distance between pillars, pillar height, total surface area, the chamber volume and the surface area to volume ratios of each chip are shown in Table 1.

**Table 1**

| **Chip number** | **1** | **2** | **3** | **4** | **5** | **6** |
|---|---|---|---|---|---|---|
| **Pillar size (µm)** | 25 | 25 | 50 | 100 | 25 | 0 |
| **Distance between pillars (µm)** | 8 | 17 | 9 | 50 | 30 | - |
| **Pillar height (µm)** | 100 | 100 | 100 | 100 | 100 | 100 |
| **Total surface area (mm²)** | 451.6 (5x) | 313.2 (3.48x) | 283.9 (3.15x) | 130 (1.44x) | 220.2 (2.45x) | 90 (1x) |
| **Chamber volume (µl)** | 1.92 | 2.91 | 1.27 | 2.5 | 3.57 | 4.5 |
| **Ratio of surface area to volume** | 11.6 | 5.33 | 11.1 | 2.95 | 3 | 1 |

DNA binding efficiency and PCR efficiency with respect to the six chips prepared were determined. To measure DNA binding efficiency, a buffer was used as follows. A mixture of 1 M of sodium sulfate (pH 3.0) and 1 M of sodium acetate (pH 3.0) was used as the binding buffer, and a mixture of 0.01 M of sodium sulfate (pH 3.0) and 0.01 M of sodium acetate (pH 3.0) was used as the washing buffer. First, a sample including DNA was mixed with the binding buffer in a ratio of 1:1, and the mixture was added to a chip, and subsequently the chip was washed by adding the washing buffer to the chip. The amount of DNA in the binding buffer and the washing buffer eluted from the chip was quantified using picogreen (Molecular Probe), and the amount of DNA bound to the chip was predicted by the difference between the DNA amount quantified in the buffers and the amount of DNA existing in the initial sample.

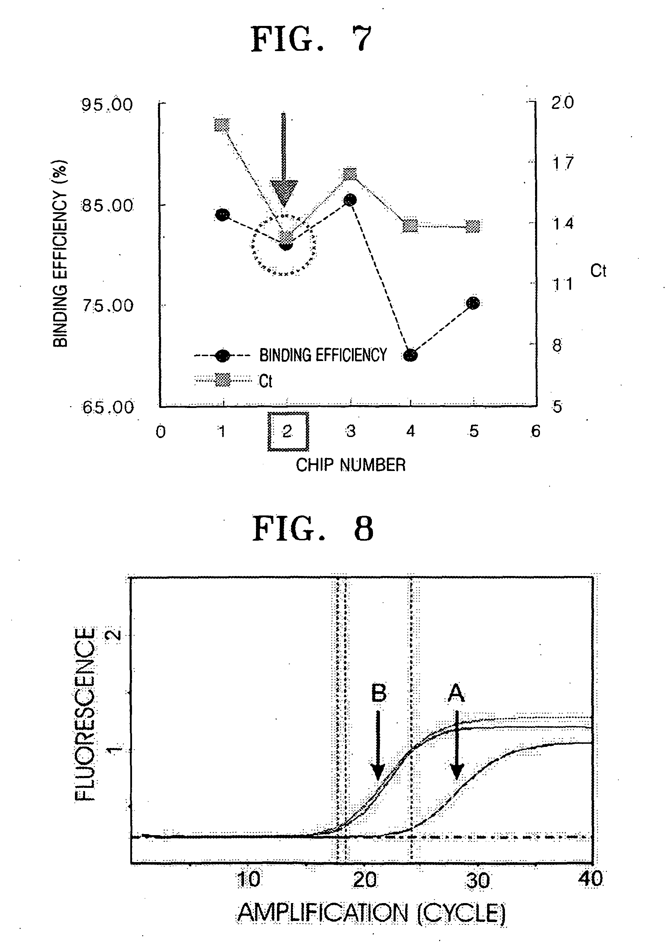

PCR was performed in the same manner as in Example 1, except that 5 % of PEG and 0.62 mg/ml of BSA were added to the PCR mixture with respect to each chip. FIG. 7 is a graph showing the DNA binding efficiency and the PCR efficiency according to the 6 chips having different surface areas, according to an embodiment of the present invention. As shown in FIG. 7, chip no. 2, having a pillar size of 25 µm and a 17 µm distance between the pillars of, has the lowest Ct value and a high DNA binding efficiency (represented by an arrow in FIG. 7). From this results, it can be seen that DNA binding efficiency is generally proportional to a surface area of a chip, and the larger the surface area is, the higher is the DNA binding efficiency. However, the larger the surface area of a chip, the higher the Ct value, thus the lower the PCR efficiency.

### Example 5: Concentration and amplification efficiency of a nucleic acid using purified DNA

Concentration and amplification efficiency of a nucleic acid was determined using purified 0.012 ng/µl of *E. coli* BL21 gDNA (16700 target gene copy/µl). In the step of DNA concentration, a mixture of 1 M of sodium sulfate (pH 3.0) and 1 M of sodium acetate (pH 3.0) was used as the binding buffer, and a mixture of 0.01 M of sodium sulfate (pH 3.0) and 0.01 M of sodium acetate (pH 3.0) was used as the washing buffer. PCR was performed in the same manner as in Example 4. FIG. 8 is a graph showing the PCR efficiency according to the nucleic acid concentration. In FIG. 8, curve A shows the results after performing PCR with the initial DNA solution prior to concentration. Curve B shows the PCR results of DNA, which was concentrated using the method of concentrating and amplifying a nucleic acid according to an embodiment of the present invention. As shown in FIG. 8, the Ct value of curve B is significantly lower than the Ct value prior to concentration (curve A). Therefore, when the method of concentrating and amplifying a nucleic acid according to an embodiment of the present invention is used, DNA is efficiently concentrated on a chip.

To quantitatively estimate the efficiency of concentrating a nucleic acid using the method of concentrating and amplifying a nucleic acid according to an embodiment of the present invention, the Ct values depending on the copy number of *E. coli* BL21 gDNA as a template were measured. The results are shown in Table 2.

**Table 2**

| **DNA (target gene copy/chip)** | **Ct** |
|---|---|
| 3.00E+06 | 14.86 |
| 1.00E+06 | 16.77 |
| 3.33E+05 | 18.22 |
| 1.11 E+05 | 20.80 |
| 3.70E+0₄ | 22.32 |
| 1.23E+04 | 24.23 |

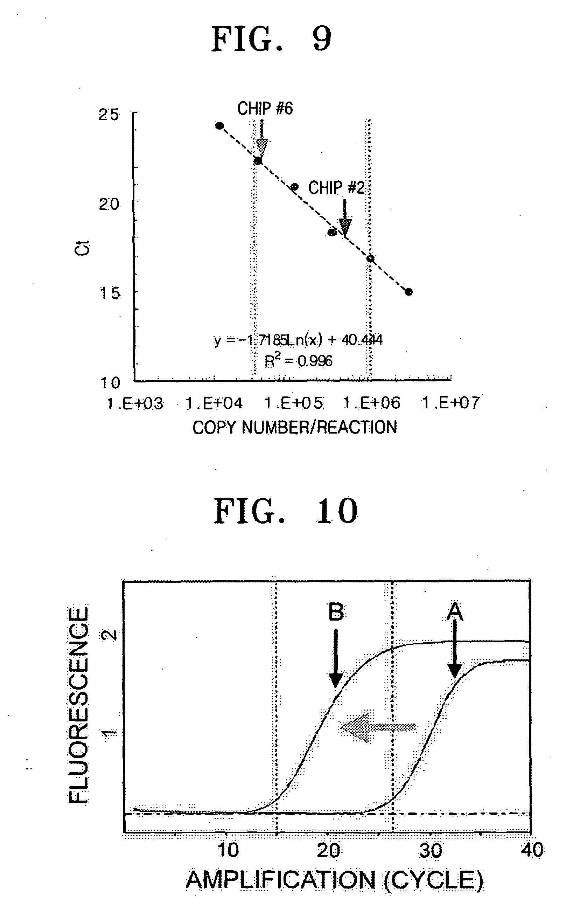

Using the data shown in Table 2, a standard curve that represents Ct values over the copy number of the target gene of E. coli BL21 gDNA was plotted. FIG. 9 is the standard curve showing Ct values according to the copy number of the target genes of *E. coli* BL21 gDNA, according to an embodiment of the present invention. In FIG. 9, the dotted line on the right refers to the Ct value corresponding to *E. coli* BL21 gDNA, which is 40 times concentrated without losses (1.00E+06 copy) compared to the initial DNA. The dotted line on the left refers to the Ct value corresponding to the original BL21 gDNA solution (16700 copy/µl) prior to concentration. As shown in FIG. 9, using chip no. 6, which has no pillar structure, the concentration efficiency of DNA is so low that the corresponding Ct value is relatively high. Contrary thereto, using chip no. 2, the concentration efficiency of DNA is very high so that the Ct value is very low. The Ct-value of chip no. 2 represents a Ct value corresponding to 35 % of 1.00E+06 copy. Accordingly, it can be seen that losses of 65 % occurred in a process of 40 times concentration, but a considerable amount of DNA was concentrated.

### Example 6: Concentration and amplification efficiency of a nucleic acid using cell lysate

The concentration and amplification efficiency of a nucleic acid were determined using 1.3×10⁵ cell/µl of *E. coli* BL21 lysate as the sample. In the step of DNA concentration, a mixture of 1 M of sodium sulfate (pH 3.0) and 1 M of sodium acetate (pH 3.0) was used as the binding buffer, and a mixture of 0.01 M of sodium sulfate (pH 3.0) and 0.01 M of sodium acetate (pH 3.0) was used as the washing buffer. PCR was performed in the same manner as in Example 4. FIG. 10 is a graph showing the PCR efficiency according to the nucleic acid concentration, according to an embodiment of the present invention. In FIG. 10, curve A represents the results of performing PCR from an initial DNA solution prior to concentration. Curve B represents the PCR results obtained when DNA was concentrated using the method of concentrating and amplifying a nucleic acid according to an embodiment of the present invention. Referring to FIG. 10, it can be seen that the Ct value prior to concentration was 25.5, but the Ct value after nucleic acid concentration significantly decreased to 15.37. That is, when the method of concentrating and amplifying a nucleic acid according to an embodiment of the present invention is used, the nucleic acid can be efficiently concentrated, purified and amplified even from cell lysate, in which a large amount of PCR inhibitor exists.

To quantitatively estimate the nucleic acid concentration efficiency using the method of concentrating and amplifying a nucleic acid according to an embodiment of the present invention, the Ct value corresponding to predetermined cell number of *E. coli* BL21 used as a template was measured. The results are shown in Table 3.

**Table 3**

| **Cell number** | **Ct** |
|---|---|
| 1.00E+07 | 19.69 |
| 3.33E+06 | 21.24 |
| 1.11 E+06 | 22.72 |
| 3.70E+05 | 24.74 |
| 1.23E+05 | 26.37 |
| 4.12E+04 | 27.80 |

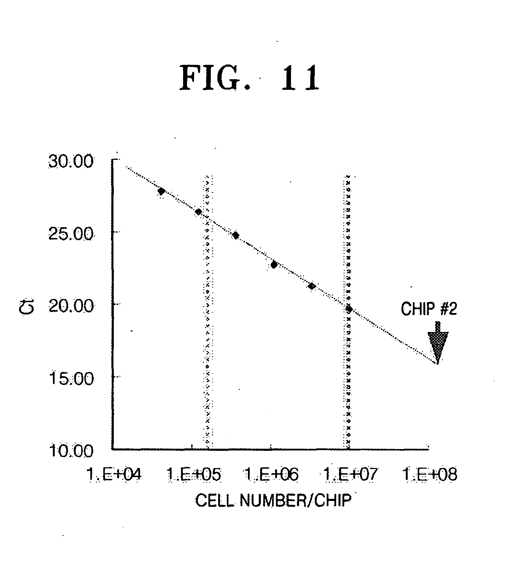

Using the data shown in Table 3, a standard curve that represents the Ct value over the cell number of *E. coli* BL21 was plotted. FIG. 11 is the standard curve showing the Ct value subject to the cell number of *E. coli* BL21, according to an embodiment of the present invention. In FIG. 11, the dotted line on the right refers to the Ct value (19.69) corresponding to *E. coli* BL21 cells, which are 50 times (1 × 10⁷ cell/µl) concentrated in respect to the initial sample. The dotted line on the left refers to the Ct value corresponding to the cell number of the original BL21 sample (1.3×10⁵ cell/µl) prior to concentration. Referring to FIG. 11, using chip no. 2, the Ct value obtained is significantly lower than 19.69. When the Ct value corresponding to chip no. 2 is converted to the corresponding cell number according to the standard curve, it represents more than about 500 times concentration of an original cell sample. This is because PCR inhibitors existing in cell lysate were removed in the process of concentrating the nucleic acid and thus, the nucleic acid was purified in purer form.

Therefore, when the method of concentrating and amplifying a nucleic acid according to an embodiment of the present invention is used, nucleic acids of cell lysates including PCR inhibitors can be efficiently purified, concentrated and amplified.

According to the present invention, since the nucleic acid is reversibly bound on the surface of the micro chamber, the PCR recovery yield is higher compared with a surface composed of aluminium oxide, to which nucleic acids irreversible bind. In addition, all the processes are sequentially performed in a single micro chamber so that the number of samples and consumables can be reduced, time and labor consumed in the treatment and analysis can be reduced, detection sensitivity can be improved since sample losses due to transferring the sample from one process to the other is avoided, and the danger of cross contamination can be significantly reduced. Therefore, establishing a complete automated system for concentration and amplification of a nucleic acid can be easily achieved.
## Claims

1. A method of sequentially concentrating and amplifying a nucleic acid in a single micro chamber, the method comprising the steps of:
introducing a nucleic acid-containing sample and a solution comprising a kosmotropic salt, or a mixture of said sample and said solution, into a micro chamber having a hydrophilic surface;
concentrating the nucleic acid by binding the nucleic acid to the surface of the micro chamber;
adding a PCR mixture to the micro chamber; and
amplifying the nucleic acid by performing a polymerase chain reaction (PCR).

2. The method of claim 1, further comprising the step of washing the micro-chamber after concentrating the nucleic acid and prior to performing PCR.

3. The method of claim 1, comprising the further step of adding polyethylene glycol and/or bovine serum albumin to the micro chamber prior to performing PCR.

4. The method of claim 3, wherein the concentrations of the polyethylene glycol and bovine serum albumin are 1-10 % by volume and 0.1-10 mg/ml, respectively.

5. The method of claim 1, wherein the hydrophilic functional group is selected from the group consisting of a hydroxyl group, an amine group, a carboxyl group, a polycarboxyl group and a silanol group.

6. The method of claim 1, wherein the kosmotropic salt is selected from the group consisting of sulfate (SO42-), hydrogenphosphate (HPO42-), hydroxide (OH-), fluoride (F-), formate (HCOO-) and acetate (CH₃COO-).

7. The method of claim 1, wherein the nucleic acid-containing sample and the solution comprising a kosmotropic salt have a pH of 3-10.

8. The method of claim 1, wherein the concentration of the kosmotropic salt is 100-2000 mM.

9. The method of claim 1, wherein the nucleic acid-containing sample is blood, serum, urine, saliva or cell lysate of bacteria existing in a cell culture fluid.

## Patentansprüche

1. Verfahren zum aufeinander folgenden Konzentrieren und Amplifizieren einer Nukleinsäure in einer Einzelmikrokammer, wobei das Verfahren die folgenden Schritte umfasst:
Einbringen einer Nukleinsäure enthaltenden Probe und einer Lösung, die ein kosmotropes Salz umfasst, oder einer Mischung aus der Probe und der Lösung, in eine Mikrokammer mit einer hydrophilen Oberfläche;
Konzentrieren der Nukleinsäure durch Binden der Nukleinsäure an die Oberfläche der Mikrokammer;
Zugeben einer PCR-Mischung zu der Mikrokammer; und
Amplifizieren der Nukleinsäure durch Ausführen einer Polymerasekettenreaktion (PCR).

2. Verfahren nach Anspruch 1, außerdem umfassend den Schritt des Waschens der Mikrokammer nach dem Konzentrieren der Nukleinsäure und vor dem Ausführen der PCR.

3. Verfahren nach Anspruch 1, umfassend den weiteren Schritt des Zugebens von Polyethylenglycol und/oder Rinderserumalbumin zu der Mikrokammer vor dem Ausführen der PCR.

4. Verfahren nach Anspruch 3, wobei die Konzentrationen des Polyethylenglycols und Rinderserumalbumins 1-10 Vol.-% bzw. 0,1-10 mg/ml betragen.

5. Verfahren nach Anspruch 1, wobei die hydrophile funktionelle Gruppe ausgewählt ist aus der Gruppe bestehend aus einer Hydroxylgruppe, einer Amingruppe, einer Carboxylgruppe, einer Polycarboxylgruppe und einer Silanolgruppe.

6. Verfahren nach Anspruch 1, wobei das kosmotrope Salz ausgewählt ist aus der Gruppe bestehend aus Sulfat (SO₄²⁻), Hydrogenphosphat (HPO₄²⁻), Hydroxid (OH⁻), Fluorid (F⁻), Formiat (HCOO⁻) und Acetat (CH₃COO⁻).

7. Verfahren nach Anspruch 1, wobei die Nukleinsäure enthaltende Probe und die Lösung, die ein kosmotropes Salz umfasst, einen pH von 3-10 aufweisen.

8. Verfahren nach Anspruch 1, wobei die Konzentration des kosmotropen Salzes 100-2000 mM beträgt.

9. Verfahren nach Anspruch 1, wobei es sich bei der Nukleinsäure enthaltenden Probe um Blut, Serum, Urin, Speichel oder ein Zelllysat von Bakterien handelt, die in einer Zellkulturflüssigkeit vorkommen.

## Revendications

1. Procédé de concentration et d'amplification successives d'un acide nucléique dans une seule microchambre, ledit procédé comprenant les étapes consistant à :
introduire un échantillon contenant un acide nucléique et une solution comprenant un sel cosmotrope, ou un mélange dudit échantillon et de ladite solution, dans une microchambre possédant une surface hydrophile ;
concentrer l'acide nucléique par liaison de l'acide nucléique à la surface de la microchambre ;
ajouter un mélange pour PCR à la microchambre ; et
amplifier l'acide nucléique par mise en oeuvre d'une réaction en chaîne de la polymérase (PCR).

2. Procédé selon la revendication 1, comprenant en outre l'étape de lavage de la microchambre après la concentration de l'acide nucléique et avant la mise en oeuvre de la PCR.

3. Procédé selon la revendication 1, comprenant l'étape supplémentaire d'ajout de polyéthylèneglycol et/ou de sérumalbumine bovine dans la microchambre avant la mise en oeuvre de la PCR.

4. Procédé selon la revendication 3, dans lequel les concentrations du polyéthylèneglycol et de la sérumalbumine bovine sont respectivement de 1-10 % en volume et de 0,1-10 mg/ml.

5. Procédé selon la revendication 1, dans lequel le groupe fonctionnel hydrophile est choisi dans l'ensemble constitué d'un groupe hydroxyle, d'un groupe amine, d'un groupe carboxyle, d'un groupe polycarboxyle et d'un groupe silanol.

6. Procédé selon la revendication 1, dans lequel le sel cosmotrope est choisi dans le groupe constitué du sulfate (SO₄²⁻), de l'hydrogénophosphate (HPO₄²⁻), de l'hydroxyde (OH⁻), du fluorure (F⁻), du formiate (HCOO⁻) et de l'acétate (CH₃COO⁻).

7. Procédé selon la revendication 1, dans lequel l'échantillon contenant un acide nucléique et la solution comprenant un sel cosmotrope ont un pH de 3-10.

8. Procédé selon la revendication 1, dans lequel la concentration du sel cosmotrope est de 100-2000 mM.

9. Procédé selon la revendication 1, dans lequel l'échantillon contenant un acide nucléique est du sang, du sérum, de l'urine, de la salive ou un lysat cellulaire de bactéries présentes dans un fluide de culture cellulaire.
